(19) Europäisches Patentamt
European Patent Office
Office européen des brevets

(11) Numéro de publication: **0 232 659 B1**

# (12) FASCICULE DE BREVET EUROPEEN

(45) Date de publication de fascicule du brevet: **21.08.91**

(51) Int. Cl.[5]: **C07D 471/04**, A61K 31/435, //(C07D471/04,221:00,221:00)

(21) Numéro de dépôt: **86402948.3**

(22) Date de dépôt: **29.12.86**

Le dossier contient des informations techniques présentées postérieurement au dépôt de la demande et ne figurant pas dans le présent fascicule.

## (54) Nouvelles phényl naphtyridines comportant un substituant méthyle en position 3, et les médicaments qui en contiennent.

(30) Priorité: **06.01.86 FR 8600102**
**06.01.86 FR 8600104**

(43) Date de publication de la demande: **19.08.87 Bulletin 87/34**

(45) Mention de la délivrance du brevet: **21.08.91 Bulletin 91/34**

(84) Etats contractants désignés: **AT BE CH DE ES FR GB GR IT LI LU NL SE**

(56) Documents cités:
**EP-A- 0 000 490**
**EP-A- 0 018 735**
**EP-A- 0 172 058**
**US-A- 4 128 649**
**US-A- 4 215 123**

(73) Titulaire: **LABORATOIRES UPSA**
**1 bis, rue du Docteur Camille Bru**
**F-47000 Agen(FR)**

(72) Inventeur: **Teulon, Jean-Marie**
**13, Avenue Guibert**
**F-78170 La Celle St Cloud(FR)**

(74) Mandataire: **Portal, Gérard et al**
**Cabinet Beau de Loménie 55, rue d'Amsterdam**
**F-75008 Paris(FR)**

EP 0 232 659 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

Rank Xerox (UK) Business Services

## Description

La présente invention concerne les phényl-naphtyridines de formule (1). Elle concerne également le procédé de préparation desdits produits et leurs applications, notamment en thérapeutique.

Les nouveaux composés selon l'invention sont choisis parmi l'ensemble constitué par les composés de formule générale (1);

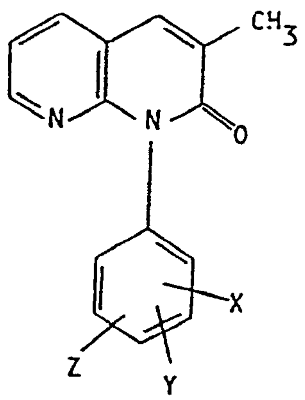

(I)

dans laquelle :

x, Y et Z sont choisis parmi les combinaisons suivantes :

a) Y = Z = H et X est : m-$CH_3$, m-Cl, m-$SCH_3$, m,o,p-f, m,p-CN, m-$CONH_2$, m-$CH_2$-CN ;

b) Z = H et X et Y sont fluor plus particulièrement en position 2,5 ou 2,4 ou 2,6 ;

c) Z = H, X = m-CN et Y est p-Cl ou p-F ;

pour la préparation de médicaments, notamment anti-ulcères et/ou anti-sécrétoires.

La Demanderesse a déjà décrit des produits de la famille des phényl-naphtyridines dans FR-A-2 567 520 ou dans EP-A-0 172 058 présentant une activité anti-ulcères et/ou anti-sécrétoires ; mais elle a maintenant découvert, de façon inattendue, que certains produits spécifiques de ladite famille présentaient des propriétés anti-ulcères et/ou anti-sécrétoires améliorées et/ou un meilleur rapport thérapeutique que les produits de même famille décrits et exemplifiés dans ladite demande.

Ainsi par exemple, lorsque le noyau aromatique ne comporte qu'une substitution, cette substitution, lorsqu'elle est $CH_3$, $SCH_3$, CN, $CH_2CN$ ou $CONH_2$, doit se situer en position méta, lorsqu'elle est Cl, elle peut être en position méta ou para, alors que, lorsqu'elle est F, elle peut se situer en position méta, ortho ou para.

Ainsi par exemple, il est avantageux que les deux substituants du noyau aromatique soient F ; dans ce cas, les trois combinaisons 2,6 2,5 et 2,4 sont possibles mais les positions 2,5 et 2,4 sont préférables. La combinaison 3-CN 4-halogène (F ou Cl) est également particulièrement souhaitable.

Les composés de formule (I) selon l'invention peuvent être synthétisés :

- soit par une réaction de Réformatsky suivie d'une déshydratation,
- soit par une réaction de Wittig suivie d'une déshydratation,
- soit par une réaction de Perkin,
- soit par une réaction de Claisen,

sur un aldéhyde de formule (II):

Ainsi par exemple, lorsque le noyau aromatique ne comporte qu'une substitution, cette substitution, lorsqu'elle est $CH_3$, $SCH_3$, CN, $CH_2CN$ ou $CONH_2$, doit se situer en position méta, lorsqu'elle est Cl, elle peut être en position méta ou para, alors que, lorsqu'elle est F, elle peut se situer en position méta, ortho ou para.

Ainsi par exemple, il est avantageux que les deux substituants du noyau aromatique soient F ; dans ce cas, les trois combinaisons 2,6 2,5, et 2,4 sont possibles mais les positions 2,5 et 2,4 sont préférables. La combinaison 3-CN 4-halogène (F ou Cl) est également particulièrement souhaitable.

Les composés de formule (I) selon l'invention peuvent être synthétisés :

- soit par une réaction de Réformatsky suivie d'une déshydratation,
- soit par une réaction de Wittig suivie d'une cyclisation,
- soit par une réaction de Perkin,
- soit par une réaction de Claisen,

sur un aldéhyde de formule (II):

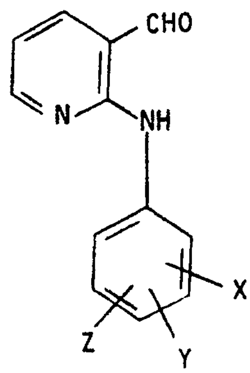

(II)

dans la formule (II), X, Y et Z sont définis comme ci-dessus.

D'une façon générale, les aldéhydes de formule (II) peuvent être obtenus, par oxydation d'un alcool de formule (III) à l'aide d'un oxydant doux tel que par exemple $MnO_2$, dans un solvant organique comme le dichlorométhane ou le chloroforme, à une température comprise entre 20 et 50° C.

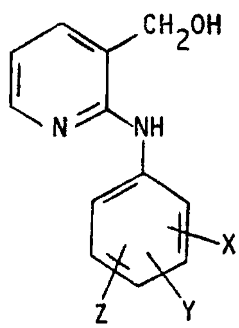

(III)

Dans la formule (III), X, Y et Z sont définis comme ci-dessus.

Les alcools de formule (III) sont obtenus par réduction classique telle que par exemple à l'hydrure double d'aluminium et de lithium dans un solvant organique tel que par exemple le tétrahydrofuranne d'un acide ou d'un de ses esters de formule (IV). Dans le cas où noyau phényle sera porteur d'un substitution sensible à certains réducteurs telle que par exemple nitrile, on choisira la réduction de l'ester par un réducteur respectant cette substitution par exemple le borohydrure de lithium préparé "in situ" à partir du borohydrure de potassium et du chlorure de lithium.

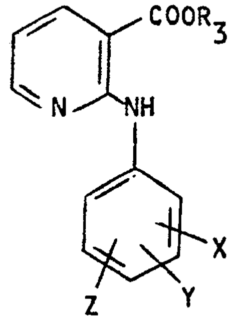

(IV)

Dans la formule (IV) X, Y et Z sont définis comme ci-dessus. $R_3$ est l'atome d'hydrogène ou un alkyle.

Le procédé préféré industriellement pour la synthèse des produits de formule (I) consiste dans l'utilisation de la réaction connue de Perkin par laquelle on fait réagir un aldéhyde de formule (II) sur un anhydride de formule :

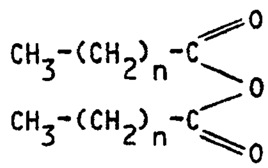

en présence du sel de sodium de l'acide correspondant.

La réaction peut être réalisée sans solvant ou dans un solvant tel que la méthylpyrrolidone à une température de 100 à 200 °C environ.

Dans ce cas précis, nous aurons n = 1 : anhydride propionique et pripionate de sodium.

Un autre procédé avantageux industriellement consiste dans l'utilisation de la réaction connue de Claisen par laquelle on fait réagir un aldéhyde de formule (II) avec des esters d'acide de formule :

$R\text{-}CH_2\text{-}COOR'$

(dans le cas présent R = $CH_3$, R′ étant un alkyle) en présence d'un alcoolate de sodium ou de potassium, ou d'hydrure de sodium ou de sodium métal. On utilisera par exemple le propionate d'éthyle et l'éthylate de sodium.

Les produits dans lesquels X ou Y ou Z est un amide sont obtenus par transformation, au moyen de procédés connus, des produits correspondants dans lesquels X ou Y ou Z possède une fonction nitrile, par exemple l'action d'un acide.

Les produits dans lesquels X ou Y ou Z est un hydroxy sont obtenus par transformation, au moyen de procédés connus des produits correspondants dans lesquels X ou Y ou Z est un alcoxy, par exemple par action du chlorhydrate de pyridine ou de l'acide bromhydrique.

Selon l'invention, on propose des compositions thérapeutiques, utiles notamment pour le traitement des ulcères gatro-intestinaux, caractérisées en ce qu'elles renferment, en association avec un excipient acceptable, au moins un composé de formule (I).

D'autres caractéristiques et avantages de l'invention seront mieux compris à la lecture qui va suivre de quelques exemples de préparation nullement limitatifs mais donnés à titre d'illustration.

Le tableau I ci-après donne la formule développée de certains produits.

Exemple 1

(méthyl-3 phényl)amino-2 hydroxyméthyl-3 pyridine

Formule III X = 3 - $CH_3$ ; Y = Z = H

A une suspension de 9,6 g d'hydrure double d'aluminium et de lithium dans 230 ml d'éther anhydre, on ajoute goutte à goutte une solution de 37,5 g d'acide (méthyl-3 phényl)amino-2 nicotinique dans 140 ml de tétrahydrofuranne anhydre. Après la fin de l'addition, le mélange réactionnel est agité durant 3 h à température ambiante. Après refroidissement, l'excès d'hydrure double est détruit par addition d'acétate d'éthyle puis d'une solution aqueuse saturée de sulfate de sodium. Le précipité formé est essoré et lavé à l'éther. Les filtrats rassemblés sont évaporés sous vide et on récupère 34,5 g de (méthyl-3 phényl)amino-2 hydroxyméthyl-3 pyridine sous forme d'une huile utilisée brute dans l'étape suivante.

Selon cette méthode sont préparés les dérivés suivants :

- (chloro-3 phényl)amino-2 hydroxyméthyl-3 pyridine,
  Formule III X = 3-Cl ; Y = Z = H
  Cristaux F = 114-5 °C ; rendement : 94 %
- (méthylthio-3 phényl)amino-2 hydroxyméthyl-3 pyridine
  Formule III X = 3-$SCH_3$ ; Y = Z = H
  Huile ; rendement : 95 %
- (difluoro-2,4 phényl)amino-2 hydroxyméthyl-3 pyridine
  Formule III X = 2-F ; Y = 4-F, Z = H
  Huile ; rendement : 93 %
- (fluoro-3 phényl)amino-2 hydroxyméthyl-3 pyridine
  Formule III X = 3-F ; Y = Z = H
  Cristaux F = 77-8 °C ; rendement 95 %
- (fluoro-4 phényl)amino-2 hydroxyméthyl-3 pyridine

Formule III X = 4-F ; Y = Z = H
Cristaux F = 89-90°C ; rendement 88 %

- (fluoro-2 phényl)amino-2 hydroxyméthyl-3 pyridine
  Formule III X = 2-F ; Y = Z = H
  Cristaux F = 96-8°C ; rendement 95 %
- (difluoro-2,5 phényl)amino-2 hydroxyméthyl-3 pyridine
  Formule III X = 2-F ; Y = 5-F ; Z = H
  Cristaux F- 71-4°C ; rendement 98 %
- (difluoro-2,6 phényl)amino-2 hydroxyméthyl-3 pyridine
  Formule III X = 2-F ; Y = 6-F ; Z = H
  Cristaux F = 115°C ; rendement 90 %
- (chloro-4 phényl)amino-2 hydroxyméthyl-3 pyridine
  Formule III X = 4-Cl ; Y = Z = H
  Cristaux F = 124-126°C ; rendement 95 %
- (méthoxy-4 phényl)amino-2 hydroxyméthyl-3 pyridine
  Formule III X = 4-$OCH_3$ ; Y = Z = H
  Cristaux F = 95-6°C ; rendement 95 %
- (triméthoxy-3,4,5 phényl)amino-2 hydroxyméthyl-3 pyridine
  Formule III X = 3-$OCH_3$ ; Y = 4-$OCH_3$ ; Z = 5-$OCH_3$
  Cristaux F = 127°C ; rendement 95 %

Exemple 2
(cyano-3 phényl)amino-2 hydroxyméthyl-3 pyridine
Formule III X = 3-CN ; Y = Z = H

A une solution de 39,3 g de (cyano-3 phényl)amino-2 nicotinate de méthyle dans 600 ml de tétrahydrofuranne contenant 10 g de borohydrure de potassium, on ajoute par petites quantités, sous agitation, 8 g de chlorure de lithium. Après la fin de l'addition, le mélange est porté au reflux durant 4 h puis concentré sous vide. Après addition d'eau et de glace au résidu obtenu, on extrait à l'éther et la phase éthérée est lavée à l'eau puis séchée sur sulfate de sodium. Après évaporation de l'éther, on obtient 31,6 g de (cyano-3 phényl)amino-2 hydroxyméthyl-3 pyridine sous forme de cristaux de point de fusion 126°C.

Selon ce procédé sont préparés les dérivés suivants :

- (cyano-4 phényl)amino-2 hydroxyméthyl-3 pyridine
  Formule III X = 4-CN ; Y = Z = H
  Cristaux F = 142°C ; rendement 93 %
- (cyanométhyl-3 phényl)amino-2 hydroxyméthyl-3 pyridine
  Formule III X = 3-$CH_2$-CN ; Y = Z = H
  Huile ; rendement 35 %
- (cyano-3 chloro-4 phényl)amino-2 hydroxyméthyl-3 pyridine
  Formule III X = 3-CN ; Y = 4-Cl ; Z = H
  Cristaux F = 147°C ; rendement 90 %
- (cyano-3 fluoro-4 phényl)amino-2 hydroxyméthyl-3 pyridine
  Formule III X = 3-CN ; Y = 4-F ; Z = H
  Cristaux F = 126°C ; rendement 90 %

Exemple 3
(méthyl-3 phényl)amino-2 nicotinaldéhyde
Formule II X = 3-$CH_3$ ; Y = Z = H

A une solution de 34,5 g de (méthyl-3 phényl)amino-2 hydroxyméthyl-3 pyridine préparés à l'exemple 1 dans 550 ml de chloroforme, on ajoute par petites fractions 163 g de $MnO_2$. Après la fin de l'addition, on agite à température ambiante durant 6 h.

Le milieu réactionnel est ensuite filtré sur célite et le filtrat est évaporé à sec. Les cristaux obtenus alors sont recristallisés dans l'éther isopropylique. On récupère ainsi 27 g de (méthyl-3 phényl)amino-2 nicotinaldéhyde sous forme de cristaux de point de fusion 95-7°C.

Selon cette méthode sont préparés les dérivés suivants :

- (chloro-3 phényl)amino-2 nicotinaldéhyde
  Formule II X = 3-Cl ; Y = Z = H

Cristaux F = 99-100° C ; rendement 78 %

- (méthylthio-3 phényl)amino-2 nicotinaldéhyde
  Formule II X = 3-$SCH_3$ Y = Z = H
  Cristaux F = 63-4° C ; rendement 70 %
- (difluoro-2,4 phényl)amino-2 nicotinaldéhyde
  Formule II X = 2-F ; Y = 4-F ; Z = H
  Cristaux (isopropanol) F = 135-7° C ; rendement 75 %
- (fluoro-3 phényl)amino-2 nicotinaldéhyde
  Formule II X = 3-F ; Y = Z = H
  Cristaux F = 70-72° C ; rendement 78 %
- (fluoro-4 phényl)amino-2 nicotinaldéhyde
  Formule II X = 4-F ; Y = Z = H
  Cristaux F = 67-68° C ; rendement 71 %
- (fluoro-2 phényl)amino-2 nicotinaldéhyde
  Formule II X = 2-F ; Y = Z = H
  Cristaux F = 93-94° C ; rendement 70 %
- (difluoro-2,5 phényl)amino-2 nicotinaldéhyde
  Formule II X = 2-F ; Y = 5-F ; Z = H
  Cristaux (isopropanol) F = 129-130° C ; rendement 76 %
- (difluoro-2,6 phényl)amino-2 nicotinaldéhyde
  Formule II X = 2-F ; Y = 6-F ; Z = H
  Cristaux F = 106° C ; rendement 93 %
- (cyano-3 phényl)amino-2 nicotinaldéhyde
  Formule II X = 3-CN ; Y = Z = H
  Cristaux (acétonitrile) F = 153-154° C ; rendement 60 %
- (cyano-4 phényl)amino-2 nicotinaldéhyde
  Formule II X = 4-CN ; Y = Z = H
  Cristaux F = 166° C ; rendement 93 %
- (cyanométhyl-3 phényl)amino-2 nicotinaldéhyde
  Formule II X = 3-$CH_2$-CN ;Y = Z = H
  Cristaux F = 50° C ; rendement 92 %
- (cyano-3 chloro-4 phényl)amino-2 nicotinaldéhyde
  Formule II X = 3-CN ; Y = 4-Cl ; Z = H
  Cristaux (acétonitrile) F = 203° C ; rendement 60 %
- (cyano-3 fluoro-4 phényl)amino-2 nicotinaldéhyde
  Formule II X = 3-CN ; Y = 4-F ; Z = H
  Cristaux (acétonitrile) F = 193° C ; rendement 80 %
- (chloro-4 phényl)amino-2 nicotinaldéhyde
  Formule II X = 4-Cl ; Y = Z = H
  Cristaux (acétate d'isopropyle) F = 101-102° C ; rendement 60 %
- (triméthoxy-3,4,5 phényl)amino-2 nicotinaldéhyde
  Formule II X = 3-$OCH_3$ ; Y = 4-$OCH_3$ ; Z = 5-$OCH_3$
  Cristaux F = 104° C ; rendement 25 %
- (méthoxy-4 phényl)amino-2 nicotinaldéhyde
  Formule II X = 4-$OCH_3$ ; Y = Z = H
  Cristaux F = 82-84° C ; rendement 50 %

Exemple 4

(méthyl-3 phényl)-1 dihydro-1,2 oxo-2 méthyl-3 naphtyridine-1,8

Formule I X = 3-$CH_3$ ; Y = Z = H

On porte au reflux durant 1 h 15 min un mélange de 10 g de (méthyl-3 phényl)amino-2 nicotinaldéhyde synthétisés à l'exemple 3, de 6,2 g de propionate de sodium et de 12 ml d'anhydride propionique

Le mélange réactionnel est ensuite refroidi, additionné de 100 ml d'eau et agité 30 min à température ambiante. On extrait au dichlorométhane, la phase organique est lavée à l'eau, avec une solution de soude 10 % puis encore à l'eau, séchée sur sulfate de sodium et concentrée sous vide. Le résidu obtenu cristallise. Après recristallisation dans l'isopropanol, on obtient 4,8 g de (méthyl-3 phényl)-1 dihydro-1,2 oxo-2 méthyl-3 naphtyridine-1,8 sous forme de cristaux de point de fusion 182-3° C.

Selon cette méthode ont été préparés les dérivés suivants :

Exemple 5
(chloro-3 phényl)-1 dihydro-1,2 oxo-2 méthyl-3 naphtyridine-1,8
Formule I X = 3-Cl, Y = Z = H
Cristaux (acétonitrile) F = 205-6° C ; rendement 48 %

Exemple 6
(méthylthio-3 phényl)-1 dihydro-1,2 oxo-2 méthyl-3 naphtyridine-1,8
Formule I X = 3-$SCH_3$ ; Y = Z = H
Cristaux (méthanol) F = 184-5° C ; rendement 42 %

Exemple 7
(difluoro-2,4 phényl)-1 dihydro-1,2 oxo-2 méthyl-3 naphtyridine-1,8
Formule I X = 2-F ; Y = 4-F ; Z = H
Cristaux (isopropanol) F = 188-9° C ; rendement 49 %

Exemple 8
(fluoro-3 phényl)-1 dihydro-1,2 oxo-2 méthyl-3 naphtyridine-1,8
Formule I X = 3-F ; Y = Z = H
Cristaux (diméthylformamide) F = 240-1° C ; rendement 42 %

Exemple 9
(fluoro-4 phényl)-1 dihydro-1,2 oxo-2 méthyl-3 naphtyridine-1,8
Formule I X = 4-F ; Y = Z = H
Cristaux (diméthylformamide) F = 248-9° C ; rendement 50 %

Exemple 10
(fluoro-2 phényl)-1 dihydro-1,2 oxo-2 méthyl-3 naphtyridine-1,8
Formule I X = 2-F ; Y = Z = H
Cristaux (isopropanol) F = 161-3° C ; rendement 40 %

Exemple 11
(difluoro-2,5 phényl)-1 dihydro-1,2 oxo-2 méthyl-3 naphtyridine-1,8
Formule I X = 2-F ; Y = 5-F ; Z = H
Cristaux (isopropanol) F = 143-4° C ; rendement 52 %

Exemple 12
(difluoro-2,6 phényl)-1 dihydro-1,2 oxo-2 méthyl-3 naphtyridine-1,8
Formule I X = 2-F ; Y = 6-F ; Z = H
Cristaux (éthanol) F = 212-3° C ; rendement 52 %

Exemple 13
(cyano-3 phényl)-1 dihydro-1,2 oxo-2 méthyl-3 naphtyridine-1,8
Formule I X = 3-CN ; Y = Z = H
Cristaux (diméthylformamide) F = 266-267° C ; rendement 70 %

Exemple 14
(cyano 4 phényl)-1 dihydro-1,2 oxo-2 méthyl-3 naphtyridine-1,8
Formule I X = 4-CN ; Y = Z = H
Cristaux (diméthylformamide) F = 265-267° C ; rendement 42 %

Exemple 15
(cyanométhyl-3 phényl)-1 dihydro-1,2 oxo-2 méthyl-3 naphtyridine-1,8
Formule 1 X = 3-$CH_2$-CN ; Y = Z = H

Cristaux (filtration sur gel de silice, éluant dichlorométhane 98 acétone 2) F = 174-176° C ; rendement

15 %

Exemple 16

(cyano-3 chloro-4 phényl)-1 dihydro-1,2 oxo-2 méthyl-3 naphtyridine-1,8

Formule 1 X = 3-CN ; Y = 4-C1 ; Z = H

Cristaux (acétonitrile) F = 244-5° C ; rendement 55 %

Exemple 17

(cyano-3 fluoro-4 phényl)-1 dihydro-1,2 oxo-2 méthyl-3 naphtyridine-1,8

Formule 1 X = 3-CN ; Y = 4-F ; Z = H

Cristaux (acétonitrile) F = 234° C ; rendement 57.5 %

Exemple 18

(méthoxy-4 phényl)-1 dihydro-1,2 oxo-2 méthyl-3 naphtyridine-1,8

Formule 1 X = 4-$OCH_3$ ; Y = Z = H

Cristaux (diméthylformamide) F = 235-6° C ; rendement 51 %

Exemple 19

(cyano-3 phényl)-1 dihydro-1,2 oxo-2 méthyl-3 naphtyridine-1,8

Formule 1 X = 3-CN ; Y = Z = H

On agite à température ambiante durant 4 h une solution de 10 g de (cyano-3 phényl)amino-2 nicotinaldéhyde préparés à l'exemple 3, de 20 ml de propionate et de 20 ml d'éthanol contenant 0.054 mol d'éthylate de sodium (préparé en ajoutant 1.25 g de sodium dans 20 ml d'éthanol).

Le précipité formé est essoré,lavé à l'eau et séché. Après recristallisation dans le diméthylformamide, on récupère 5,3 g de (cyano-3 phényl)-1 dihydro-1,2 oxo-2 méthyl-3 naphtyridine-1,8 sous forme de cristaux de point de fusion 266-267° C.

Exemple 20

(carbamoyl-3 phényl)-1 dihydro-1,2 oxo-2 méthyl-3 naphtyridine-1,8

Formule 1 X = 3-$CONH_2$ ; Y = Z = H

3,5 g de la (cyano-3 phényl)-1 dihydro-1,2 oxo-2 méthyl-3 naphtyridine-1,8 préparés aux exemples 13 et 21 sont ajoutés par petites fractions à 10 ml d'acide sulfurique concentré sous agitation. Après agitation 24 h à température ambiante, on additionne 100 ml d'eau au mélange réactionnel et on basifie avec une solution d'ammoniaque. Les cristaux formés sont essorés, soigneusement lavés à l'eau et séchés. Après recristallisation dans le diméthylformamide, on obtient 2.3 g de (carbamoyl-3 phényl)-1 dihydro-1,2 oxo-2 méthyl-3 naphtyridine-1,8 sous forme de cristaux de point de fusion 293-395° C.

TABLEAU I

5145-21

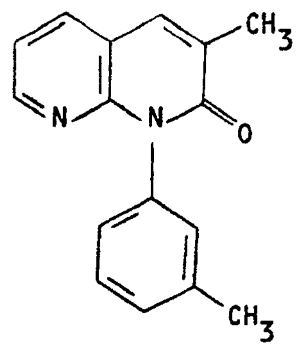

Exemple 4

5145-22

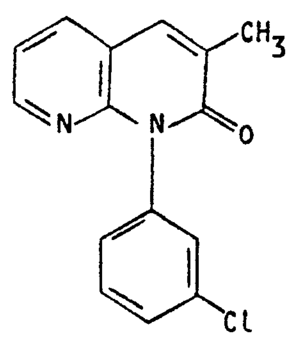

Exemple 5

5145-28

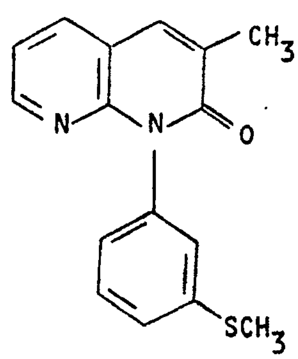

Exemple 6

5145-29

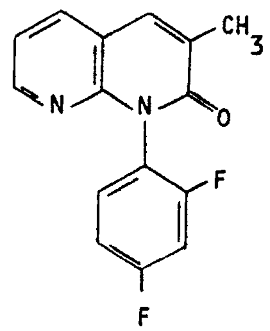

Exemple 7

5145-30

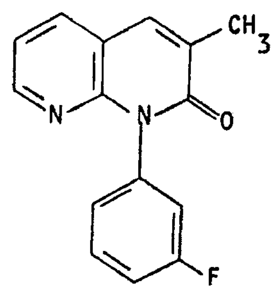

Exemple 8

5145-31

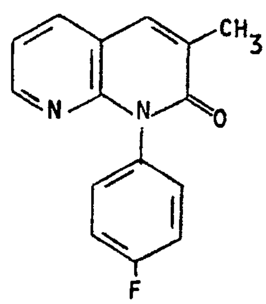

Exemple 9

5145-32

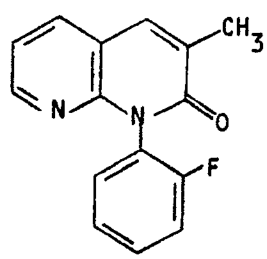

Exemple 10

5145-34

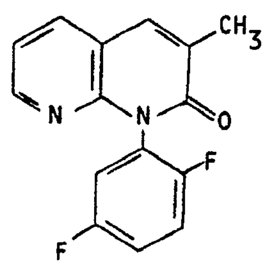

Exemple 11

5145-68 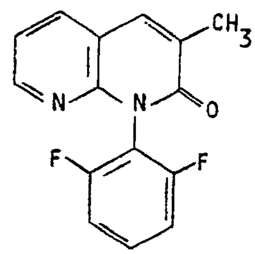 Exemple 12

5145-17 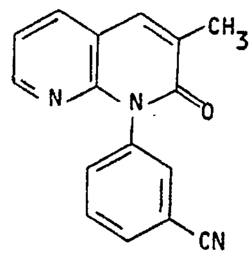 Exemple 13

5145-37 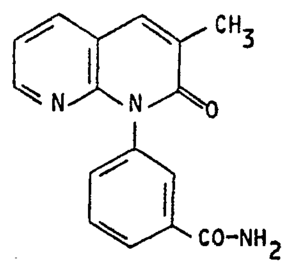 Exemple 22

5145-69 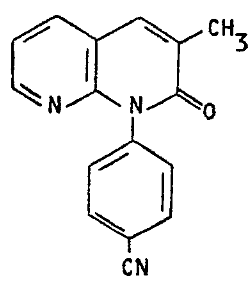 Exemple 14

5145-75 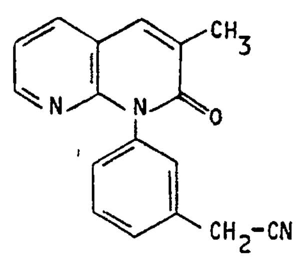 Exemple 15

5145-98 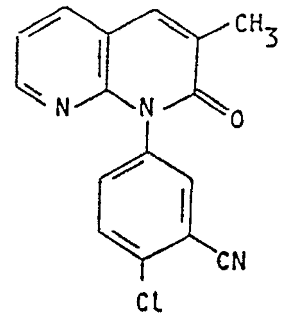 Exemple 16

5145-104 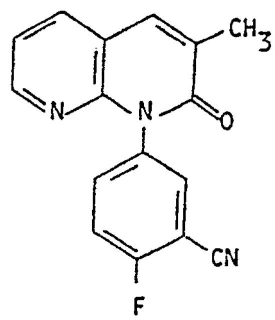 Exemple 17

PHARMACOLOGIE

Activité anti-ulcères

1. Méthode

Des lots de 10 à 30 rats mâles de souche OFA (provenance IFFA CREDO, France) pesant 160-180 g, sont mis à la diète hydrique 24 h avant l'administration orale du produit à étudier.

Trente minutes après le gavage, un agent ulcérigène médicamentaux ou un agent nécrosant (éthanol absolu) est administré par voie orale, à des doses qui entraînent chez les animaux témoins une ulcération gastrique maximale. Les estomacs sont ensuite prélevés selon le test, respectivement 6 h et 1 h après le dernier traitement. Les lésions gastriques sont alors évaluées macroscopiquement (cotation et mesure de l'ampleur des ulcères).

2. Résultats

Les résultats sont exprimés sous forme de doses actives 50 (dose inhibant de 50 % les lésions provoquées) déterminées graphiquement à partir de la droite exprimant la relation entre les pourcentages d'inhibition et les doses utilisées.

Dose Active 50

(experimée en mg. kg-1, administration par voie orale)

| | 1) Administration d'un agent médicamenteux ulcérigène | 2) Administration d'un agent nécrosant |
|---|---|---|
| Exemple 5 | 0,170 | 0,017 |
| Exemple 8 | 0,180 | 0,020 |
| Exemple 9 | 0,340 | 0,035 |
| Exemple 13 | 0,085 | 0,010 |
| Exemple 17 | 0,164 | 0,024 |

1) agent anti-inflammatoire non stéroïdien, 2) éthanol.

Activité anti-sécrétoire

1. Méthode

Une ligature au niveau du pylore est pratiquée sous anesthésie à l'éther chez des lots de 5 rats mâles de souche OFA (provenance IFFA CREDO, France) pesant 180-200 g.

Le produit à étudier est administré par voie sous cutanée au temps de la ligature. Les animaux sont sacrifiés 4 h plus tard et le liquide gastrique est recueilli puis son acidité titrée par la soude 0,1N à pH 4 et 7.

2. Résultats

Les résultats sont exprimés en doses actives 50 (dose provoquant une inhibition de 50 % de la sécrétion acide totale) déterminée graphiquement à partir de la droite exprimant le pourcentage d'inhibition de la sécrétion acide totale en fonction des doses utilisées.

Dose Active 50

(exprimée en mg.kg-1, administration par voie sous-cutanée)

| | |
|---|---|
| Exemple 5 | 0,038 |
| Exemple 8 | 0,045 |
| Exemple 9 | 0,040 |
| Exemple 13 | 0,050 |
| Exemple 17 | 0,064 |

TOXICOLOGIE

Les premières études toxicologiques réalisées chez le rat à jeun Sprague Dawley, après administration par voie orale, ont permis de montrer que les $DL_{50}$ pour les produits exemplifiés sont supérieures ou égales à 300 mg.kg-1.

CONCLUSION

Les produits selon l'invention et leurs sels d'addition d'acides non toxiques décrits dans la présente demande semblent particulièrement intéressants ; leurs activités anti-ulcères et anti-sécrétoires sont extrêmement puissantes et supérieures à celles décrites pour les dérivés ayant fait l'objet de la demande de brevet français 2 567 520.

Ils pourront être utilisés par voie injectable ou orale dans le traitement des ulcères gastro-duodénaux, sous forme d'ampoules injectables de comprimés ou de gélules dosés de 5 à 100 mg.

**Revendications**

**Revendications pour les Etats contractants suivants : BE, CH, DE, FR, GB IT, LI, LU, NL, SE**

**1.** phényl-naphtyridines, caractérisées en ce qu'elles répondent à la formule:

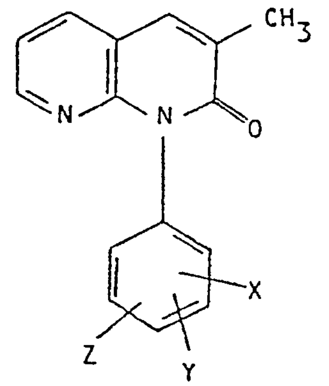

(I)

dans laquelle:

X, Y et Z sont choisis parmi les combinaisons suivantes:

a) Y = Z = H et X est : m-$CH_3$, m-Cl, m-$SCH_3$, m,o,p-F, m,p-CN, m-$CONH_2$, m-$CH_2CN$;

b) Z = H et X et Y sont fluor plus particulièrement en position 2,5 ou 2,4 ou 2,6 ;

c) Z = H, X = m-CN et Y est p-Cl ou p-F.

2. Phényl-naphtyridine selon la revendication 1, caractérisée en ce qu'elle choisie parmi les composés suivants:

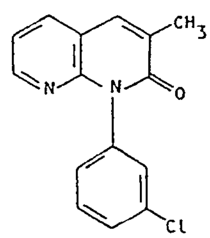

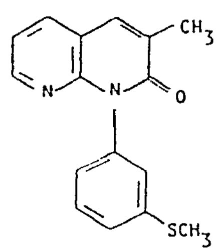

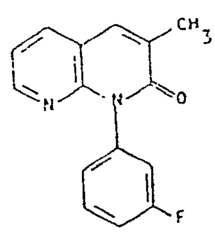

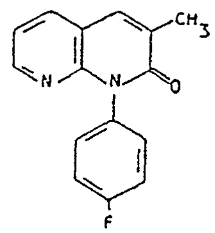

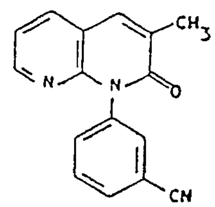

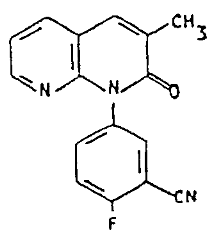

3. Médicaments utilisés notamment pour leur activité anti-ulcères et/ou anti-sécrétoires, caractérisés en ce qu'ils contiennent au moins une phényl-naphtyridine, selon l'une quelconque des revendications 1 et 2.

4. Procédé de préparation des phényl-naphtyridines de formule (I) selon l'une quelconque des revendications 1 et 2, caractérisé en ce que, selon la réaction de Perkin, on fait réagir un aldéhyde de formule (II) suivante :

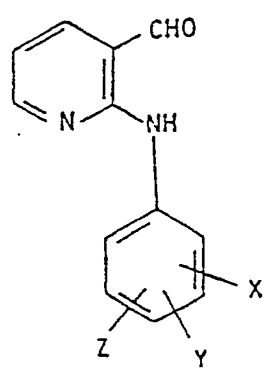

(II)

dans la formule (II) X, Y et Z sont tels que définis précédemment ; sur un anhydride de formule

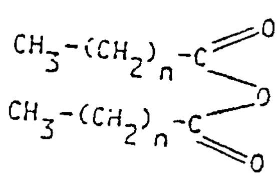

en présence du sel de sodium de l'acide correspondant, en présence ou l'absence d'un solvant.

5. Procédé selon la revendication 4, caractérisé en ce que la réaction est réalisée dans un solvant, par exemple N-méthylpyrolidone, à une température de 100 à 200° C.

6. Procédé selon la revendication 5, caractérisé en ce que n = 1, de sorte que l'on utilise l'anhydride propionique et le propionate de sodium.

7. Procédé de préparation des composés de formule (I) selon l'une quelconque des revendications 1 à 3, caractérisé en ce que, selon la réaction de Claisen, on fait réagir un aldéhyde de formule (II) avec des esters d'acide de formule :

$R-CH_2-COOR'$

dans laquelle R est un alkyle, et R' est un alkyle.

8. Procédé selon la revendication 7, caractérisé en ce que la réaction est réalisée en présence d'un alcoolate de sodium ou de potassium, ou d'hydrure de sodium ou de sodium métal.

9. Procédé selon les revendications 7 et 8, caractérisé en ce que R représente $CH_3$.

10. Procédé selon la revendication 4 ou 8, caractérisé en ce que les aldéhydes de formule (II) sont obtenus par oxydation d'un alcool de formule (III) à l'aide d'un oxydant doux dans un solvant organique à une température comprise entre 20 et 50° ;

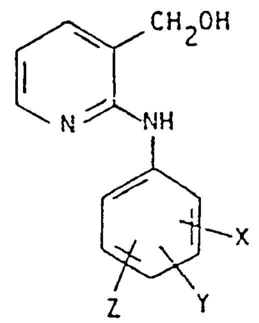

(III)

dans la formule (III) X, Y et Z sont tels que définis précédemment.

11. Procédé selon la revendication 10, caractérisé en ce que la réaction est réalisée à l'aide de $MnO_2$ comme oxydant doux et dans le dichlorométhane ou dans le chloroforme comme solvant organique.

12. Procédé selon la revendication 10, caractérisé en ce que les alcools de formule (III) sont obtenus par réduction, dans un solvant organique, d'un acide ou d'un de ses esters de formule (IV) ; dans le cas où le noyau phényle sera porteur d'une substitution sensible à certains réducteurs, on choisira la réduction de l'ester par un réducteur respectant cette substitution ;

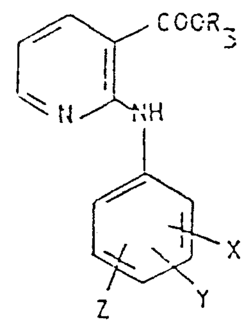

(IV)

dans la formule (IV), X, Y et Z sont tels que définis précédemment et $R_3$ est l'atome d'hydrogène ou un

alkyle.

13. Procédé selon la revendication 12, caractérisé en ce que le solvant organique est le tétrahydrofuranne, la substitution sensible est un groupe nitrile et le réducteur respectant cette substitution le borohydrure de lithium préparé "in situ" à partir du borohydrure de potassium et du chlorure de lithium.

**Revendications pour les Etats contractants suivants : AT, GR, ES**

1. Utilisation des phényl-naphtyridines de formule :

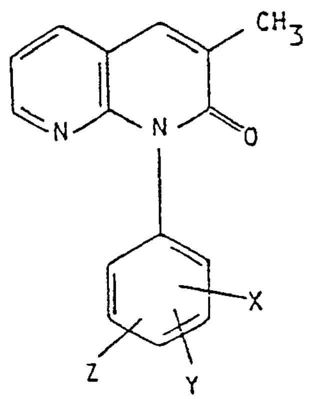

(I)

dans laquelle :
X, Y et Z sont choisis parmi les combinaisons suivantes :
a) Y = Z = H et X est : m-$CH_3$, m-Cl, m-$SCH_3$, m,o,p-F, m,p-CN, m-$CONH_2$, m-$CH_2$-CN ;
b) Z = H et X et Y sont fluor plus particulièrement en position 2,5 ou 2,4 ou 2,6 ;
c) Z = H, X = m-CN et Y est p-Cl ou p-F ;
pour la préparation de médicaments, notamment anti-ulcères et/ou anti-sécrétoires.

2. Utilisation pour la préparation de médicaments, selon la revendication 1, caractérisée en ce que la phényl-naphtyridine est choisie parmi les composés suivants :

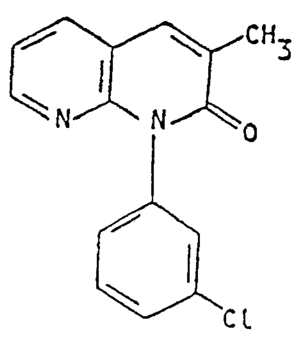

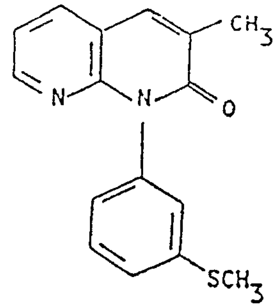

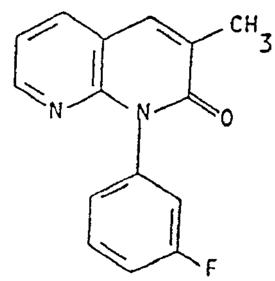

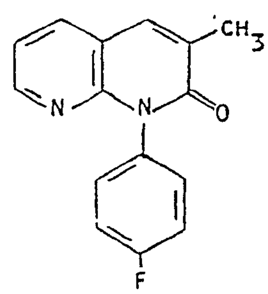

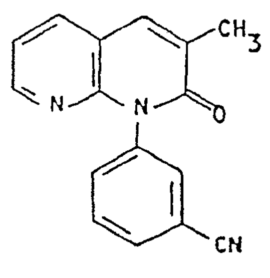

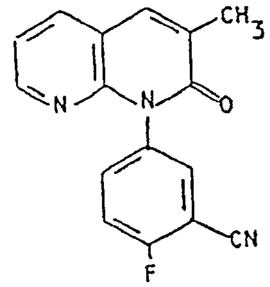

**3.** Procédé de préparation d'un médicament utilisé notament pour son activité anti-ulcères et/ou anti-sécrétoires, caractérisé en ce qu'il comprend le mélange d'au moins une phényl-naphtyridine selon l'une quelconque des revendications 1 et 2 avec un excipient ou un véhicule physiologiquement acceptable.

4. Procédé de préparation des phényl-naphtyridines de formule (I) selon l'une quelconque des revendications 1 et 2, caractérisé en ce que, selon la réaction de Perkin, on fait réagir un aldéhyde de formule (II) suivante :

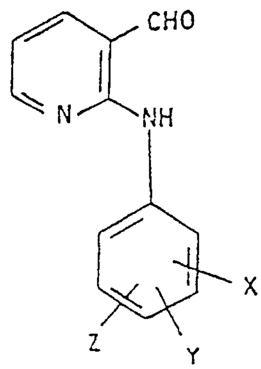

(II)

dans la formule (II) X, Y et Z sont tels que définis précédemment ; sur un anhydride de formule

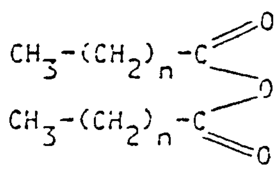

en présence du sel de sodium de l'acide correspondant, en présence ou l'absence d'un solvant.

5. Procédé selon la revendication 4, caractérisé en ce que la réaction est réalisée dans un solvant, par exemple N-méthylpyrolidone, à une température de 100 à 200° C.

6. Procédé selon la revendication 5, caractérisé en ce que n = 1, de sorte que l'on utilise l'anhydride propionique et le propionate de sodium.

7. Procédé de préparation des composés de formule (I) selon l'une quelconque des revendications 1 et 2, caractérisé en ce que, selon la réaction de Claisen, on fait réagir un aldéhyde de formule (II) avec des esters d'acide de formule :

$R\text{-}CH_2\text{-}COOR'$

dans laquelle R est un alkyle, et R' est un alkyle.

8. Procédé selon la revendication 7, caractérisé en ce que la réaction est réalisée en présence d'un alcoolate de sodium ou de potassium, ou d'hydrure de sodium ou de sodium de métal.

9. Procédé selon les revendications 7 et 8, caractérisé en ce que R représente $CH_3$.

10. Procédé selon la revendication 4 ou 8, caractérisé en ce que les aldéhydes de formule (II) sont obtenus par oxydation d'un alcool de formule (III) à l'aide d'un oxydant doux dans un solvant organique à une température comprise entre 20 et 50° C ;

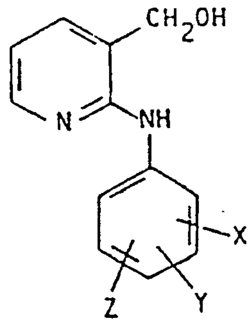

(III)

dans la formule (III) X, Y et Z sont tels que définis précédemment.

**11.** Procédé selon la revendication 10, caractérisé en ce que la réaction est réalisée à l'aide de $MnO_2$ comme oxydant doux et dans le dichlorométhane ou dans le chloroforme comme solvant organique.

**12.** Procédé selon la revendication 10, caractérisé en ce que les alcools de formule (III) sont obtenus par réduction, dans un solvant organique, d'un acide ou d'un de ses esters de formule (IV) ; dans le cas où le noyau phényle sera porteur d'une substitution sensible à certains réducteurs, on choisira la réduction de l'ester par un réducteur respectant cette substitution ;

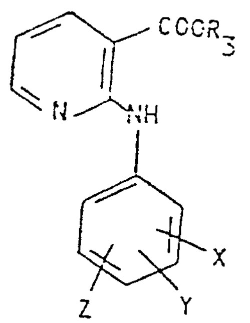

(IV)

dans la formule (IV), X, Y et Z sont tels que définis précédemment et $R_3$ est l'atome d'hydrogène ou un alkyle.

**13.** Procédé selon la revendication 12, caractérisé en ce que le solvant organique est le tétrahydrofuranne, la substitution sensible est un groupe nitrile et le réducteur respectant cette substitution le borohydrure de lithium préparé "in situ" à partir du borohydrure de potassium et du chlorure de lithium.

**Claims**

**Claims for the following Contracting States : BE, CH, DE, FR, GB IT, LI, LU, NL, SE.**

**1.** Phenylnaphthyridines, characterized in that they are of the formula:

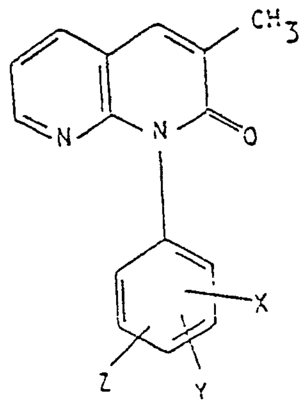

(I)

in which:

X, Y and Z are selected from the following combinations:

a) Y = Z = H and X is m-$CH_3$, m-Cl, m-$SCH_3$, m,o,p-F, m,p-CN, m-$CONH_2$, m-$CH_2$-CN;

b) Z = H and X and Y are fluorine, more particularly in the 2,5, 2,4 or 2,6 positions,

c) Z = H, X = m-CN and Y is p-Cl or p-F.

**2.** Phenylnaphthyridine according to claim 1, characterized in that it is selected from the following compounds:

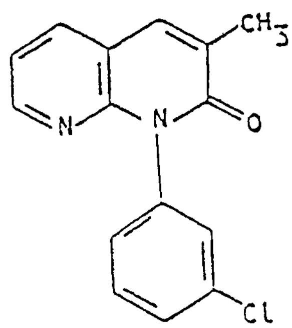

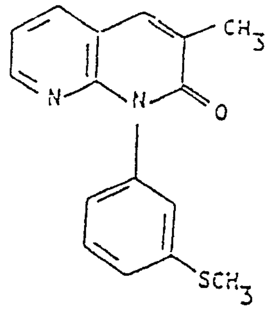

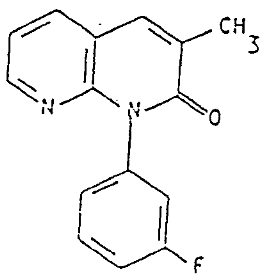

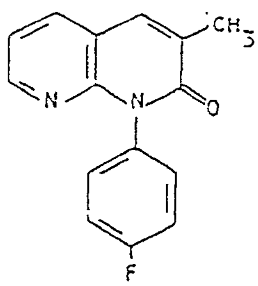

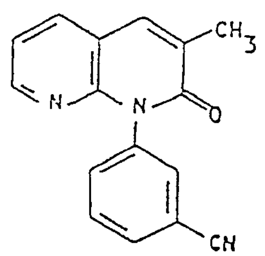

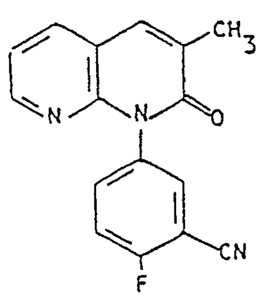

3. Drugs used in particular for their ulcer-inhibiting and/or antisecretory activity, characterized in that they comprise at least a phenylnaphthyridine according to any one of claims 1 and 2.

4. Process for the preparation of the phenylnaphthyridinesof the formula (I) according to any one of claims

1 and 2, characterized in that according to Perkin's reaction, it consists in reacting an aldehyde of the following formula (II):

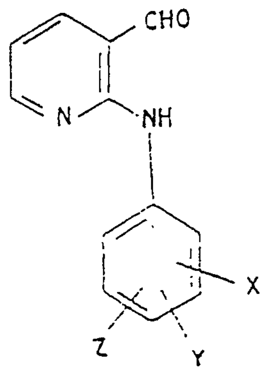

(II)

in the formula (II), X, Y and Z are as defined above, with an anhydride of the formula:

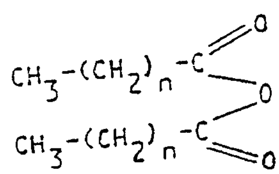

in the presence of the sodium salt of the corresponding acid and in the presence or absence of a solvent.

5. Process according to claim 4, characterized in that the reaction is carried out in a solvent, for example N-methylpyrrolidone, at a temperature of about 100 to 200 °C.

6. Process according to claim 5, characterized in that n = 1, i.e. propionic anhydride and sodium propionate are used.

7. Process for the preparation of the compounds of the formula (I) according to any one of claims 1 and 2, characterized in that, according to the Claisen reaction, an aldehyde of the formula (II) is reacted with acid esters of the formula:

$R-CH_2-COOR'$

in which R is an alkyl, and R' is an alkyl.

8. Process according to claim 7, characterized in that the reaction is carried out in the presence of a sodium or potassium alcoholate, sodium hydride or sodium metal.

9. Process according to claims 7 and 8, characterized in that R is $CH_3$.

10. Process according to claim 4 or 8, characterized in that the aldehydes of the formula (II) are obtained by the oxidation of an alcohol of the formula (III) with a mild oxidizing agent, in an organic solvent, at a temperature of between 20 and 50 °C,

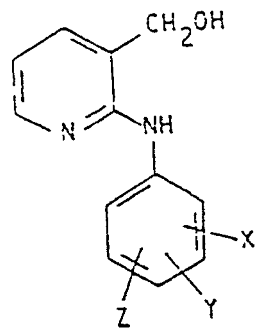

(III)

in the formula (III), X, Y and Z are as defined above.

**11.** Process according to claim 10, characterized in that the reaction is carried out with $MnO_2$ as mild oxidizing agent and in methylene chloride or in chloroform as organic solvent.

**12.** Process according to claim 10, characterized in that the alcohols of the formula (III) are obtained by reduction in an organic solvent of an acid or of one of its esters of the formula (IV); in the case where the phenyl nucleus carries a substition which is sensitive to certain reducing agents, the reducing agent for reducing the ester will be chosen so as not to affect this substitution;

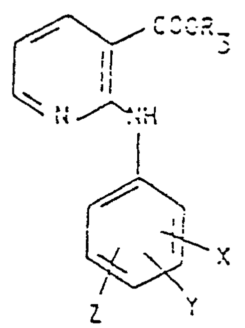

(IV)

in the formula (IV), X, Y and Z are as defined above and $R_3$ is the hydrogen atom or an alkyl.

**13.** Process according to claim 12, characterized in that the organic solvent is tetrahydrofuran, the sensitive substitution is a nitrile group and the reducing agent which does not affect this substitution is lithium borohydride prepared "in situ" from potassium borohydride and lithium chloride.

**Claims for the following Contracting States : AT, GR, ES**

**1.** Use of phenylnaphthyridines of the formula:

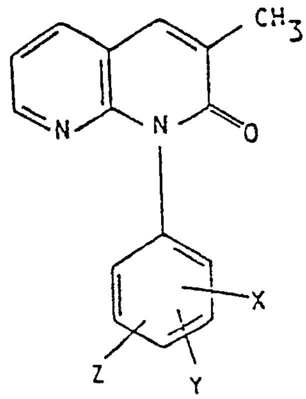

(I)

in which:

X, Y and Z are selected from the following combinations:

a) Y = Z = H and X is m-$CH_3$, m-Cl, m-$SCH_3$, m,o,p-F, m,p-CN, m-$CONH_2$, m-$CH_2$-CN;

b) Z = H and X and Y are fluorine, more particularly in the 2,5, 2,4 or 2,6 positions,

c) Z = H, X = m-CN and Y is p-Cl or p-F,

for the preparation of drugs, notably ulcer-inhibiting and/or antisecretory drugs.

2. Use for the preparation of drugs according to claim 1, characterized in that the phenylnaphthyridine is selected from the following compounds:

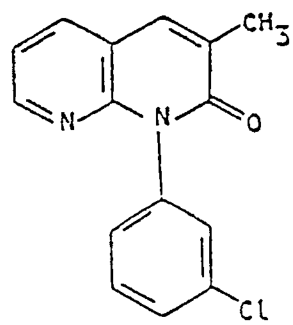

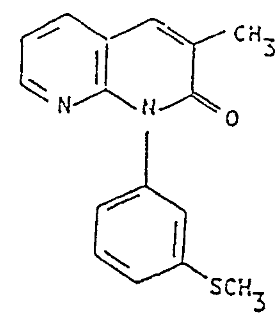

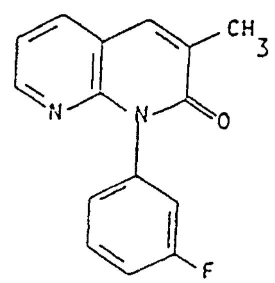

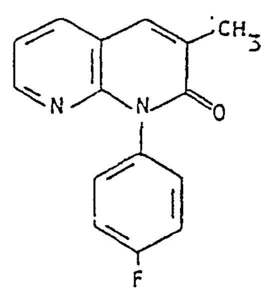

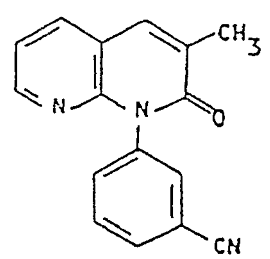

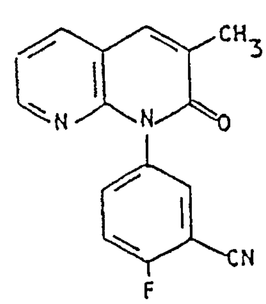

**3.** Process for the preparation of a drug used in particular for its ulcer-inhibiting and/or antisecretory activity, characterized in that it comprises the admixture of at least a phenylnaphthyridine according to any one of claims 1 and 2, with a physiologically acceptable excipient or vehicle.

4. A process for the preparation of the phenylnaphthyridines of the formula (I) according to any one of claims 1 and 2, characterized in that according to Perkin's reaction, it consists in reacting an aldehyde of the following formula (II):

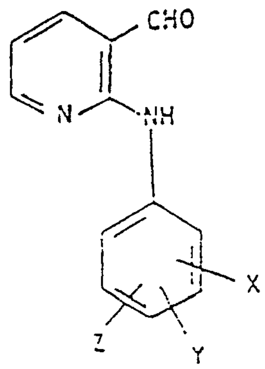

(II)

in the formula (II), X, Y and Z are as defined above, with an anhydride of the formula:

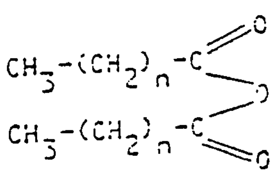

in the presence of the sodium salt of the corresponding acid and in the presence or absence of a solvent.

5. Process according to claim 4, characterized in that the reaction is carried out in a solvent, for example N-methylpyrrolidone, at a temperature of about 100 to 200 °C.

6. Process according to claim 5, characterized in that n = 1, i.e. propionic anhydride and sodium propionate are used.

7. Process for the preparation of the compounds of the formula (I) according to any one of claims 1 and 2, characterized in that, according to the Claisen reaction, an aldehyde of the formula (II) is reacted with acid esters of the formula:

$R-CH_2-COOR'$

in which R is an alkyl, and R' is an alkyl.

8. Process according to claim 7, characterized in that the reaction is carried out in the presence of a sodium or potassium alcoholate, sodium hydride or sodium metal.

9. Process according to claims 7 and 8, characterized in that R is $CH_3$.

10. Process according to claim 4 or 8, characterized in that the aldehydes of the formula (II) are obtained by the oxidation of an alcohol of the formula (III) with a mild oxidizing agent, in an organic solvent, at a temperature of between 20 and 50 °C,

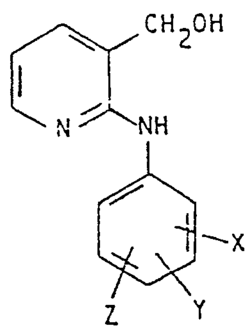

(III)

in the formula (III), X, Y and Z are as defined above.

**11.** Process according to claim 10, characterized in that the reaction is carried out with $MnO_2$ as mild oxidizing agent and in methylene chloride or in chloroform as organic solvent.

**12.** Process according to claim 10, characterized in that the alcohols of the formula (III) are obtained by reduction in an organic solvent of an acid or of one of its esters of the formula (IV); in the case where the phenyl nucleus carries a substition which is sensitive to certain reducing agents, the reducing agent for reducing the ester will be chosen so as not to affect this substitution;

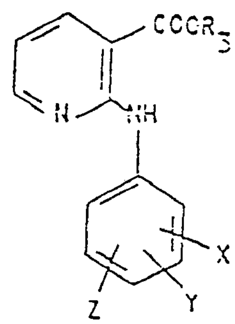

(IV)

in the formula (IV), X, Y and Z are as defined above and $R_3$ is the hydrogen atom or an alkyl.

**13.** Process according to claim 12, characterized in that the organic solvent is tetrahydrofuran, the sensitive substitution is a nitrile group and the reducing agent which does not affect this substitution is lithium borohydride prepared "in situ" from potassium borohydride and lithium chloride.

**Patentansprüche**
**Patentansprüche für folgende Vertragsstaaten : BE, CH, DE, FR, GB, IT, LI, LU, NL, SE**

**1.** Phenyl-naphthyridine, dadurch gekennzeichnet, daß sie der Formel

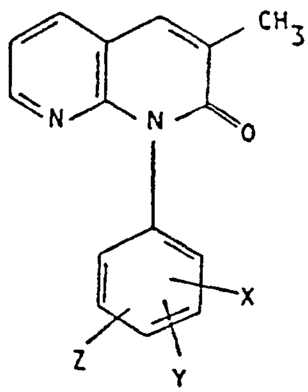

(I)

entsprechen, in welcher
X, Y und Z ausgewählt sind aus den folgenden Kombinationen:
a) Y = Z = H und X ist: m-$CH_3$, m-Cl, m-$SCH_3$, m,o,p-F, m,p-CN, m-$CONH_2$, m-$CH_2CH$;
b) Z = H und X und Y sind Fluor, genauergesagt in Position 2,5 oder 2,4 oder 2,6;
c) Z = H, X = m-CN und Y ist p-Cl oder p-F.

2. Phenyl-naphthyridin nach Anspruch 1, dadurch gekennzeichnet, daß es ausgewählt ist aus den folgenden Verbindungen

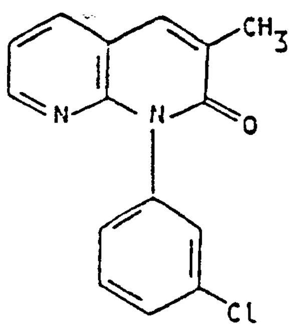

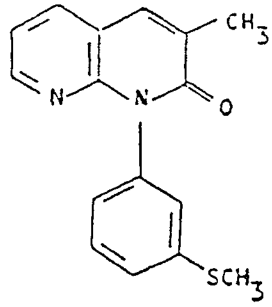

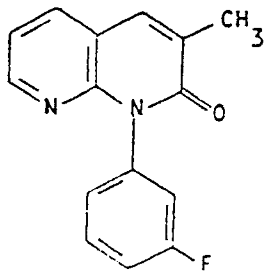

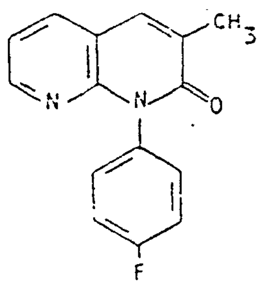

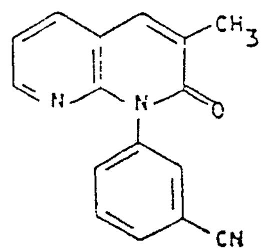

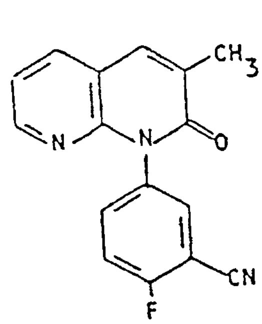

3. Medikamente, die insbesondere wegen ihrer geschwürhemmenden und/oder sekretionshemmenden Aktivität verwendet werden, dadurch gekennzeichnet, daß sie mindestens ein Phenyl-naphthyridin nach einem der Ansprüche 1 und 2 enthalten.

4. Verfahren zur Herstellung der Phenyl-naphthyridine der Formel (I) nach einem der Ansprüche 1 und 2, dadurch gekennzeichnet, daß man nach der Perkin-Reaktion ein Aldehyd der folgenden Formel (II)

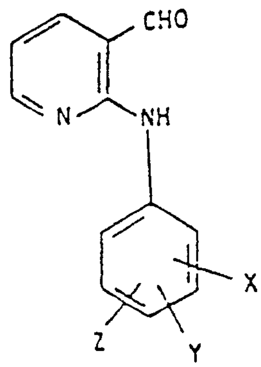

(II)

in welcher Formel (II) X, Y und Z wie zuvor definiert sind, mit einem Anyhdrid der Formel

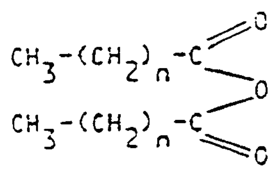

in Gegenwart des Natriumsalzes der korrespondierenden Säure in Gegenwart oder in Abwesenheit eines Lösungsmittels reagieren läßt.

5. Verfahren nach Anspruch 4, dadurch gekennzeichnet, daß die Reaktion in einem Lösungsmittel, z.B. N-Methylpyrrolidon, bei einer Temperatur von 100 bis 200° C durchgeführt wird.

6. Verfahren nach Anspruch 5, dadurch gekennzeichnet, daß n = 1, sodaß Propionsäureanhydrid und Natriumpropionat verwendet werden.

7. Verfahren zur Herstellung der Verbindungen der Formel (I) nach einem der Ansprüche 1 und 2 , dadurch gekennzeichnet, daß man nach der Claisen-Reaktion ein Aldehyd der Formel (II) mit Säureestern der Formel

$R-CH_2-COOR'$

in welcher R ein Alkyl und R' ein Alkyl ist, reagieren läßt.

8. Verfahren nach Anspruch 7, dadurch gekennzeichnet, daß die Reaktion in Gegenwart eines Natrium- oder Kaliumalkoholats oder von Natriumhydrid oder Natriummetall durchgeführt wird.

9. Verfahren nach den Ansprüchen 7 und 8, dadurch gekennzeichnet, daß R für $CH_3$ steht.

10. Verfahren nach Anspruch 4 oder 8, dadurch gekennzeichnet, daß die Aldehyde der Formel (II) durch Oxidation eines Alkohols der Formel (III) mit einem sanften Oxidationsmittel in einem organischen Lösungsmittel bei einer Temperatur zwischen 20 und 50° C erhalten werden;

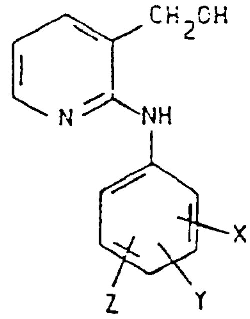

(III)

in welcher Formel (III) X, Y und Z wie zuvor definiert sind.

**11.** Verfahren nach Anspruch 10, dadurch gekennzeichnet, daß die Reaktion mit Hilfe von $MnO_2$ als sanftes Oxidationsmittel und in Dichlormethan oder in Chloroform als organisches Lösungsmittel durchgeführt wird.

**12.** Verfahren nach Anspruch 10, dadurch gekennzeichnet, daß die Alkohle der Formel (III) durch Reduktion einer Säure oder eines ihrer Ester der Formel (IV) in einem organischen Lösungmittel erhalten werden; für den Fall, daß der Phenylkern Träger einer für gewisse Reduktionsmittel empfindlichen Substitution ist, wählt man die Reduktion des Esters mittels eines diese Substitution nicht störenden Reduktionsmittels;

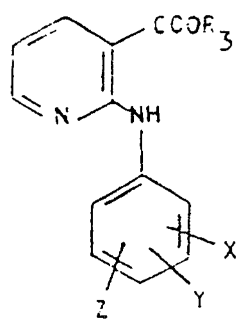

(IV)

in welcher Formel (IV) X, Y und Z wie zuvor definiert sind und R3 ein Wasserstoffatom oder ein Alkyl ist.

**13.** Verfahren nach Anspruch 12, dadurch gekennzeichnet, daß das organische Lösungsmittel Tetrahydrofuran ist, die empfindliche Substitution eine Nitrilgruppe ist und das diese Substutition nicht störende Reduktionsmittel Lithiumborhydrid ist, welches "in situ" aus Kaliumborhydrid und Lithiumchlorid hergestellt wird.

**Patentansprüche für folgende Vertragsstaaten : AT, GR, ES**

**1.** Verwendung der Phenyl-naphthyridine der Formel

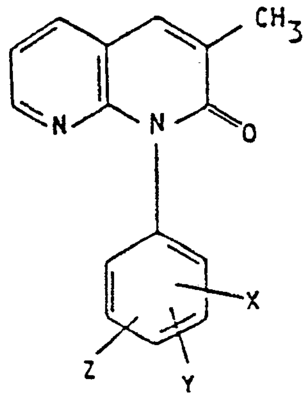

(I)

in welcher:

X, Y und Z ausgewählt sind aus den folgenden Kombinationen:

a) Y = Z = H und X ist: m-$CH_3$, m-Cl, m-$SCH_3$, m,o,p-F, m,p-CN, m-$CONH_2$, m-$CH_2$CH;

b) Z = H und X und Y sind Fluor, genauergesagt in Position 2,5 oder 2,4 oder 2,6;

c) Z = H, X = m-CN und Y ist p-Cl oder p-F;

zur Herstellung von Medikamenten, insbesondere von geschwürhemmenden und/oder sekretionshemmenden Medikamenten.

2. Verwendung zur Herstellung von Medikamenten nach Anspruch 1, dadurch gekennzeichnet, daß das Phenylnaphthyridin ausgewählt ist aus den folgenden Verbindungen:

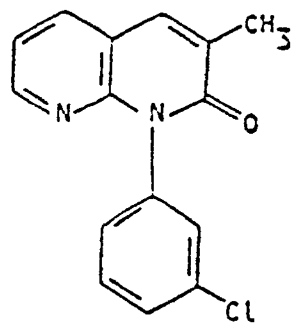

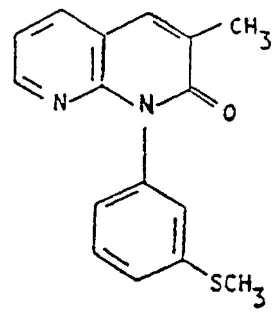

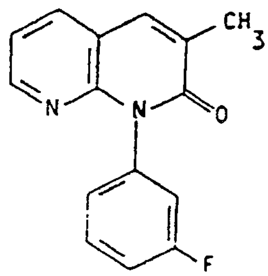

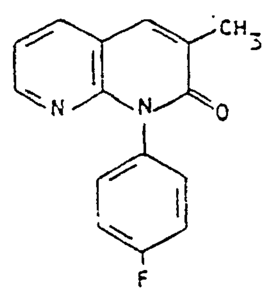

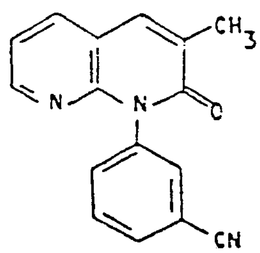

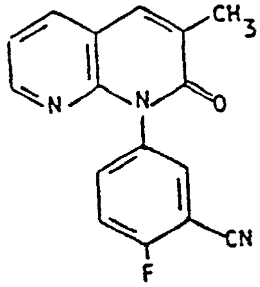

3. Verfahren zur Herstellung eines insbesondere wegen seiner geschwürhemmenden und/oder sekretionshemmenden Aktivität verwendeten Medikaments, dadurch gekennzeichnet, daß es eine Mischung aus mindestens einem Phenyl-naphthyridin nach einem der Ansprüche 1 und 2 mit einem physiologisch akzeptablen Exzipienten oder Vehikel umfaßt.

4. Verfahren zur Herstellung der Phenyl-naphthyridine der Formel (I) nach einem der Ansprüche 1 und 2, dadurch gekennzeichnet, daß man nach der Perkin-Reaktion ein Aldehyd der folgenden Formel (II)

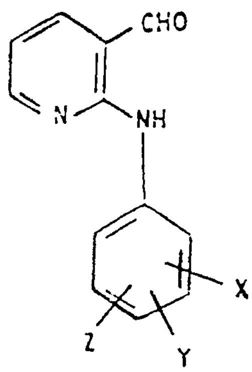

(II)

in welcher Formel (II) X, Y und Z wie zuvor definiert sind, mit einem Anyhdrid der Formel

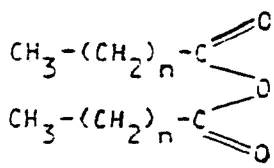

in Gegenwart des Natriumsalzes der korrespondierenden Säure in Gegenwart oder in Abwesenheit eines Lösungsmittels reagieren läßt.

5. Verfahren nach Anspruch 4, dadurch gekennzeichnet, daß die Reaktion in einem Lösungsmittel, z.B. N-Methylpyrrolidon, bei einer Temperatur von 100 bis 200° C durchgeführt wird.

6. Verfahren nach Anspruch 5, dadurch gekennzeichnet, daß n = 1, sodaß Propionsäureanhydrid und Natriumpropionat verwendet werden.

7. Verfahren zur Herstellung der Verbindungen der Formel (I) nach einem der Ansprüche 1 und 2, dadurch gekennzeichnet, daß man nach der Claisen-Reaktion ein Aldehyd der Formel (II) mit Säureestern der Formel

$R-CH_2-COOR'$

in welcher R ein Alkyl und R' ein Alkyl ist, reagieren läßt.

8. Verfahren nach Anspruch 7, dadurch gekennzeichnet, daß die Reaktion in Gegenwart eines Natrium- oder Kaliumalkoholats oder von Natriumhydrid oder Natriummetall durchgeführt wird.

9. Verfahren nach den Ansprüchen 7 und 8, dadurch gekennzeichnet, daß R für $CH_3$ steht.

10. Verfahren nach Anspruch 4 oder 8, dadurch gekennzeichnet, daß die Aldehyde der Formel (II) durch Oxidation eines Alkohols der Formel (III) mit einem sanften Oxidationsmittel in einem organischen Lösungsmittel bei einer Temperatur zwischen 20 und 50° C erhalten werden;

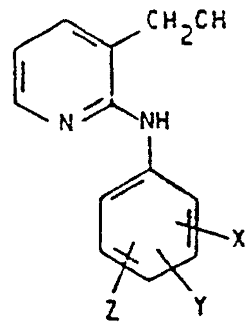

(III)

in welcher Formel (III) X, Y und Z wie zuvor definiert sind.

**11.** Verfahren nach Anspruch 10, dadurch gekennzeichnet, daß die Reaktion mit Hilfe von $MnO_2$ als sanftes Oxidationsmittel und in Dichlormethan oder in Chloroform als organisches Lösungsmittel durchgeführt wird.

**12.** Verfahren nach Anspruch 10, dadurch gekennzeichnet, daß die Alkohle der Formel (III) durch Reduktion einer Säure oder eines ihrer Ester der Formel (IV) in einem organischen Lösungmittel erhalten werden; für den Fall, daß der Phenylkern Träger einer für gewisse Reduktionsmittel empfindlichen Substitution ist, wählt man die Reduktion des Esters mittels eines diese Substitution nicht störenden Reduktionsmittels;

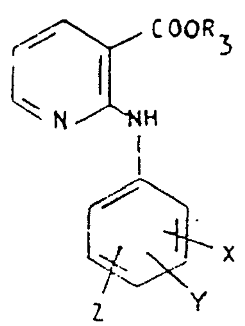

(IV)

in welcher Formel (IV) X, Y und Z wie zuvor definiert sind und R3 ein Wasserstoffatom oder ein Alkyl ist.

**13.** Verfahren nach Anspruch 12, dadurch gekennzeichnet, daß das organische Lösungsmittel Tetrahydrofuran ist, die empfindliche Substitution eine Nitrilgruppe ist und das diese Substutition nicht störende Reduktionsmittel Lithiumborhydrid ist, welches "in situ" aus Kaliumborhydrid und Lithiumchlorid hergestellt wird.